# FASCICULE DE BREVET EUROPEEN

(11) **EP 3 066 376 B1**
(45) Date de publication et mention de la délivrance du brevet: **05.06.2019**
(21) Numéro de dépôt: 13823962.9
(22) Date de dépôt: 06.11.2013
(51) Int. Cl.: F16L 43/00

(54) **DISPOSITIF DE CONNEXION ET CANALISATION L'INCORPORANT POUR TUYAUTERIE DE TRANSPORT DE FLUIDE D'UN VEHICULE AERIEN OU SPATIAL, ET PROCEDE DE FABRICATION DE CE DISPOSITIF**
VERBINDUNGSVORRICHTUNG, ROHRE DAMIT FÜR FLÜSSIGKEITSTRANSPORTROHRLEITUNGEN EINES LUFT- ODER RAUMFAHRZEUGS UND VERFAHREN ZUR HERSTELLUNG DIESER VORRICHTUNG
CONNECTION DEVICE, PIPES INCORPORATING SAME FOR FLUID TRANSMISSION PIPING OF AN AIRCRAFT OR A SPACECRAFT, AND METHOD FOR MANUFACTURING SAID DEVICE

(43) Date de publication de la demande: 14.09.2016
(73) Titulaire: Hutchinson, 75008 Paris (FR)
(72) Inventeur: GONZALEZ BAYON, Cristina, F-45200 Amilly (FR); BERGERE, Stéphane, F-45650 Saint Jean Le Blanc (FR); FLORENTZ, Bertrand, F-45200 Paucourt (FR)
(74) Mandataire: Gevers & Orès
(86) Numéro de dépôt international: PCT/FR2013/052649
(87) Numéro de publication internationale: WO 2015/067859

(56) Documents cités:
- EP-A1- 0 058 783
- EP-A1- 1 243 841
- WO-A1-2009/124910
- US-A- 6 125 891
- US-A1- 2013 220 474

## Description

La présente invention concerne un dispositif de connexion entre deux tubes et optionnellement en outre à un réservoir de fluide pour une tuyauterie de transport de ce fluide à l'intérieur d'un véhicule aérien ou spatial, une canalisation pour une telle tuyauterie incorporant ces tubes et ce dispositif de connexion les reliant entre eux, et un procédé de fabrication de ce dispositif. L'invention s'applique notamment à une tuyauterie d'un circuit de carburant d'un aéronef civil ou militaire en particulier équipé d'ailes composites, étant précisé qu'elle concerne de manière générale les connexions et fixations de toutes tubulures de transfert de fluide par exemple pour circuits de carburant, d'adduction d'eau, d'évacuation d'eaux usées, de drainage, d'oxygène ou de refroidissement, à titre non limitatif. Le document US6125891 décrit un dispositif selon l'état de la technique.

Les tuyauteries ou lignes de carburant des avions actuels sont usuellement réalisées en aluminium, à l'instar des ailes dans lesquelles elles sont logées. On commence néanmoins depuis quelques années à concevoir des ailes et des tuyauteries de carburant en matériaux composites, afin d'alléger dans la mesure du possible ces tuyauteries et la voilure l'incorporant.

Outre ce gain de masse, on a cherché à contrôler la conductivité électrique de l'ensemble de la voilure en cas de foudroiement, tout en éliminant les charges électrostatiques à l'intérieur de ces tuyauteries. En effet, il faut veiller lors d'un foudroiement à ce que, d'une part, la foudre s'écoule principalement par les structures des ailes et, d'autre part, à ce que l'accumulation de charges électrostatiques à l'intérieur de la tuyauterie parcourue par le carburant soit strictement limitée pour éviter tout risque d'explosion par inflammation du carburant.

Les embouts de connexion ou connecteurs qui permettent, d'une part, de relier entre eux des tubes composites de la tuyauterie de carburant et, d'autre part, de fixer ces tubes à la structure du réservoir de carburant au moyen d'une bride munie d'oreilles de fixation équipant ces embouts, étaient usuellement réalisés en fonderie d'aluminium par le passé. Les contraintes de réduction de poids, pour réduire la consommation et les émissions, conduisent aujourd'hui à alléger ces connecteurs en les passant en aluminium usiné, anodisé et traité pour le glissement, comme expliqué ci-après.

Pour permettre les mouvements et les déformations de fonctionnement (par exemple dues à des dilatations) relatives entre les structures et ces tubes et connecteurs, la liaison cylindrique entre le connecteur et chaque tube doit être à la fois coulissante et rotulante et elle doit présenter une rugosité faible pour faciliter ce coulissement, en réduisant ainsi les efforts de montage, les charges transmises aux structures en fonctionnement et l'usure des joints, ce qui permet d'éviter l'apparition de fuites par abrasion dans le temps au sein de la canalisation.

Pour ce faire, les liaisons cylindriques des connecteurs actuels sont usinées puis revêtues de matériaux glissants (e.g. des lubrifiants par exemple de type Molycote®, déposés par pulvérisation puis réticulés à chaud), car l'aluminium n'est pas assez glissant. De tels connecteurs métalliques sont présents sur de nombreux types de circuits aéronautiques en plus des circuits de carburant, tels que des circuits d'alimentation en eau, d'évacuation d'eaux usées, de refroidissement, de drainage, etc.

Un inconvénient majeur de ces liaisons cylindriques connues est que ces revêtements glissants déposés sur les connecteurs usinés rendent les surfaces de connexion non électriquement conductrices.

Or, compte tenu de la contrainte prioritaire précitée qui exige d'améliorer en permanence la sécurité des avions notamment pour leurs circuits de carburant logés dans des ailes en matériaux composites, il faut rechercher des nouvelles solutions qui sont à la fois suffisamment isolantes pour minimiser les risques d'explosion par foudroiement, mais également « dissipatives » pour évacuer ces charges électrostatiques.

Une deuxième contrainte majeure consiste aujourd'hui à diminuer le poids et le coût de fabrication des avions.

Grâce au changement précité de procédé de fabrication des connecteurs métalliques à base d'aluminium remplaçant l'aluminium fondu par l'aluminium usiné, anodisé et traité pour le glissement, de fortes réductions de poids ont été possible. En effet, la performance des nouveaux moyens d'usinage « UGV » combinée aux caractéristiques mécaniques très supérieures des alliages usinés dans la masse par rapport aux aluminiums moulés permettent de réduire les épaisseurs de paroi à des valeurs minimales de 1 mm, qui sont des minima technologiques pour l'usinage (car à des épaisseurs inférieures, l'effort de coupe déforme les parois), soit une masse surfacique d'environ 2,7 kg/m².

Ces connecteurs usinés et traités sont hautement conducteurs de l'électricité dans leur masse. Par contre, la tendance à la corrosion de l'aluminium nécessite de protéger ces connecteurs par des traitements de surface par exemple de type Oxydation Anodique Chromique (OAC) et également dans les zones de portée des joints d'étanchéité par des traitements de surface glissants qui, comme expliqué ci-dessus, ne conduisent pas le courant.

C'est la raison pour laquelle on utilise des tresses électriquement conductrices supplémentaires reliant, d'une part, les tubes aux connecteurs et, d'autre part, ces connecteurs à la structure du réservoir pour obtenir la conductivité électrique nécessaire pour garantir la décharge électrostatique des canalisations de carburant et éviter ainsi les risques d'explosion du réservoir de carburant par accumulation des charges électrostatiques.

Un inconvénient majeur de ces tresses conductrices est qu'elles rallongent les temps d'assemblage, les risques de mauvais montage, les coûts globaux de fabrication et d'assemblage et le poids des canalisations.

Avec l'arrivée des ailes en composite et le risque que la foudre transite préférentiellement par les tuyauteries de carburant, ces connecteurs en aluminium usiné et traité, même si leurs surfaces sont isolantes, présentent de trop fortes conductivités électriques dans leur masse pour empêcher tout passage de foudre dans les tuyauteries. La solution actuelle consiste à réaliser des « coupures-foudres » en incorporant à la tuyauterie des tubulures électriquement isolantes appelées « isolateurs ». Ces isolateurs sont typiquement aujourd'hui réalisés au moyen de structures tubulaires bobinées en fibres isolantes de verre.

Un inconvénient majeur de ces tubulures électriquement isolantes insérées entre les tubes de la tuyauterie réside dans un nombre élevée de pièces supplémentaires qui coûtent cher et alourdissent encore l'aéronef.

Le document WO-A1-2011/007100 divulgue des dispositifs de connexion entre tubes métalliques destinés à protéger une tuyauterie de carburant d'aéronef contre la foudre. Ces dispositifs incluent des connecteurs plastiques et des connecteurs métalliques reliant des tubes entre eux, étant précisé que les connecteurs plastiques comportent une enveloppe radialement externe à deux embouts entourant un embout interne, et sont réalisés en un matériau composite moulé par injection à base d'une matrice thermoplastique renforcée par des fibres de carbone discontinues de longueur par exemple comprise entre 3 et 5 mm. Des joints toriques réalisés en élastomère électriquement isolant assurent une liaison étanche entre ces connecteurs et les tubes qu'ils relient entre eux.

Un inconvénient de ces joints isolants réside dans la nécessité de rapporter des tresses métalliques sur les tubes et/ou sur les connecteurs pour permettre une continuité électrique entre connecteurs et tubes et avec la structure adjacente, de sorte à évacuer les charges électrostatiques vers cette structure.

Un but de la présente invention est de proposer un dispositif de connexion pour tuyauterie de transport de fluide d'un véhicule aérien ou spatial, le dispositif étant adapté pour relier deux tubes entre eux et optionnellement en outre à un réservoir du fluide, le dispositif comprenant un embout moulé par injection qui présente au moins une zone coudée ou incurvée et qui est constitué d'un matériau composite à matrice thermoplastique renforcée par un système de renforcement comprenant des fibres de carbone, l'embout présentant un plan de joint de moulage longitudinal médian, qui remédie notamment à l'ensemble des inconvénients précités.

A cet effet, un dispositif de connexion selon l'invention est tel que lesdites fibres de carbone s'étendent de manière orientée (i.e. non aléatoire) le long de l'embout, lequel incorpore des moyens de rigidification mécanique et vibratoire qui sont venus de moulage avec l'embout et qui s'étendent dans ledit plan de joint ou symétriquement par rapport à ce plan en ladite au moins une zone coudée ou incurvée et/ou à proximité immédiate de cette zone.

On notera que cet embout thermoplastique renforcé par ces fibres de carbone orientées de manière globalement axiale présente simultanément de hautes propriétés mécaniques et une conductivité électrique contrôlée par ces fibres (i.e. juste nécessaire à l'obtention de la conductivité électrique nécessaire pour évacuer les charges électrostatiques dans la canalisation).

On notera également que l'embout de l'invention ne pénalise pas le poids ni la tenue mécanique ou vibratoire des interfaces tubes-embout. Au contraire, cet embout permet d'alléger le dispositif de connexion et donc l'ensemble de la canalisation l'incorporant, telle qu'une canalisation de carburant, de réduire le temps de montage des pièces dans l'avion par l'intermédiaire de ce renfort, et l'intégration de fonctions supplémentaires, comme cela sera expliqué ci-après.

On notera en outre que l'utilisation d'un tel dispositif de connexion essentiellement constitué de cet embout à matrice thermoplastique était à ce jour inenvisageable dans l'industrie aéronautique, du fait :
- des propriétés mécaniques trop élevées requises, notamment la rigidité vibratoire,
- de l'impossibilité d'admettre un quelconque fluage de l'embout, inacceptable pour garantir l'étanchéité des liaisons cylindriques dans le temps, et
- de la non-conductivité électrique des thermoplastiques.

En effet, si l'on choisissait d'augmenter la conductivité électrique d'un thermoplastique en le chargeant de particules conductrices, telles qu'un noir de carbone conducteur, par exemple, le taux nécessaire de ces particules (aux environs de 20 à 30 %) serait tel que les propriétés mécaniques deviendraient alors trop faibles pour pouvoir prétendre à la substitution d'un alliage d'aluminium en vue de l'allégement de l'embout. Et si l'on choisissait plutôt d'augmenter les caractéristiques mécaniques d'un thermoplastique en le chargeant par exemple avec des fibres de verre courtes, les caractéristiques mécaniques obtenues seraient trop faibles pour concurrencer un aluminium usiné dans la masse. En somme, l'obtention de l'une de ces deux propriétés se ferait au détriment de l'autre.

En résumé, les fibres de carbone orientées renforçant l'embout de l'invention permettent d'obtenir simultanément le blocage en fluage de l'embout, l'augmentation de la rigidité vibratoire propre à alléger encore cet embout et la conductivité électrique nécessaire pour évacuer les charges électrostatiques.

De préférence, ladite matrice thermoplastique est à base d'au moins un polymère choisi dans le groupe constitué par les polyamides tels que le PA 12, les polyaryléthercétones (PAEK), les polyétheréthercétones (PEEK), les polyéthercétonecétones (PEKK) et leurs alliages, cette matrice présentant une densité de préférence inférieure ou égale à 1,5.

En prenant en compte les densités et proportions des fibres précitées et en utilisant l'un de ces polymères thermoplastiques de densité inférieure ou égale à 1,5, on obtient un matériau thermoplastique renforcé et conducteur de densité de l'ordre de 1,7 +/- 0,15 soit, pour une épaisseur de 1,4 mm, une masse surfacique inférieure à 2,4 kg/m² apportant un gain de masse de 12 %.

Selon une autre caractéristique de l'invention, ledit système de renforcement peut être présent dans l'embout selon une fraction massique comprise entre 10 % et 40 %, ce système comprenant de préférence :
- lesdites fibres de carbone, qui présentent de préférence une longueur moyenne comprise entre 0,5 mm et 3 mm et sont choisies dans le groupe constitué par celles de module intermédiaire (« IM ») ou de haut module (« HM »), et
- des fibres et/ou particules électriquement isolantes telles que des fibres de verre ou d'aramide, selon un ratio volumique fibres de carbone / fibres et/ou particules électriquement isolantes compris entre 30 % et 80 %.

Bien que les fibres de carbone « IM » ou « HM » soient particulièrement indiquées pour renforcer l'embout de l'invention, on notera que le choix du type de fibres et de la quantité se fera en réglant la conductivité diélectrique nécessaire. Avec la double contrainte de ne pas être trop conducteur tout en étant dissipatif, l'embout doit présenter une résistivité comprise entre des valeurs de l'ordre de 100 Ohm/m à 100 MOhm/m.

Comme l'on souhaite maximiser le renforcement (i.e. la rigidité et la quantité des fibres) sans pénaliser le poids de l'embout et obtenir une résistivité intermédiaire, on choisit de préférence de mélanger les fibres de carbone (électriquement conductrices) et les fibres et/ou particules électriquement isolantes suivant les ratios précités.

Selon une autre caractéristique de l'invention, ledit embout peut comporter :
- deux portions droites qui s'étendent axialement de part et d'autre de ladite au moins une zone coudée ou incurvée (i.e. n+1 portions droites pour n zones coudées ou incurvées, avec n entier ≥ 1) et qui incorporent lesdites fibres de carbone majoritairement orientées dans la direction axiale d'une surface globalement cylindrique de chaque portion (i.e. dans une direction non oblique globalement parallèle à l'axe longitudinal de symétrie de chaque portion droite), et
- au moins une collerette ou bride par exemple pour fixer l'embout audit réservoir qui est également venue de moulage avec l'embout et qui s'étend radialement de manière adjacente à ladite au moins une zone coudée ou incurvée, cette collerette ou bride incorporant lesdites fibres de carbone majoritairement orientées dans la direction radiale autour de cette portion droite (i.e. dans une direction globalement perpendiculaire à l'axe de symétrie de la bride).

On notera que ces caractéristiques d'orientation des fibres de carbone résultent de travaux de recherche et de conception approfondis de la Demanderesse pour l'obtention d'un embout qui soit apte à encaisser les efforts sans fluer (notamment aux zones d'efforts permanents comme les fixations ou attaches) et avec une orientation adéquate des fibres pour obtenir la rigidité et les gains de poids visés. La Demanderesse est ainsi parvenue à identifier des zones de l'embout où les fibres de carbone sont d'orientations similaires et donc où l'embout présente des propriétés mécaniques analogues. L'ajout des moyens de rigidification mécanique et vibratoire permet en complément d'atteindre les caractéristiques requises liées au comportement mécanique de l'embout.

Selon un mode particulier de réalisation de l'invention, lesdits moyens de rigidification mécanique et vibratoire comprennent au moins une nervure ou patte qui s'étend dans la direction axiale de l'embout et qui définit une surépaisseur radiale en ladite au moins une zone coudée ou incurvée et/ou à proximité immédiate de cette zone.

Selon un exemple de l'invention, lesdits moyens de rigidification mécanique et vibratoire comprennent au moins une dite nervure qui s'étend axialement dans ledit plan de joint de l'embout et qui prend appui sur ladite bride. Conformément à cet exemple de l'invention, ladite au moins une zone coudée ou incurvée présentant une courbure par exemple à angle droit définissant une surface externe et une surface interne des portions droites adjacentes qui sont respectivement tournées vers l'extérieur et vers l'intérieur de la courbure, lesdits moyens de rigidification mécanique et vibratoire comprennent une première dite nervure axiale formant une surépaisseur radiale sur ladite surface externe et/ou une seconde dite nervure axiale formant une surépaisseur radiale sur ladite surface interne.

On notera que ces nervures ou surépaisseurs selon l'invention peuvent être ainsi localisées à l'extrados et/ou à l'intrados dans le plan axial de « dépliage » de la zone coudée (i.e. le plan de joint de moulage), ce qui facilite le démoulage sans complexifier les moules et contribue en outre à réduire la masse de l'embout.

Selon un autre exemple de l'invention, lesdits moyens de rigidification mécanique et vibratoire comprennent deux dites pattes par exemple en forme de grilles qui s'étendent symétriquement l'une de l'autre par rapport audit plan de joint de l'embout en prenant appui sur ladite surface externe de la courbure et qui sont surmontées par une plaque de liaison audit réservoir.

On notera qu'un avantage supplémentaire du moulage par injection d'un composite à matrice thermoplastique pour obtenir l'embout est qu'il facilite l'ajout des moyens de rigidification mécanique, tels que cette ou ces nervure(s) ou pattes, aux emplacements appropriés pour obtenir la rigidité vibratoire requise, par exemple au pied des oreilles de fixation ou dans les zones des rayons de raccordement, mais également pour rigidifier la zone coudée ou incurvée qui, sous l'effet de fond de la pression, travaille en « dépliage ».

En diminuant les épaisseurs et en nervurant les zones surcontraintes, on peut alors envisager des épaisseurs d'embout de l'ordre de 1,4 mm, par exemple, pour résister à la fois aux pressions et rigidités vibratoires requises.

Avantageusement, le dispositif de connexion peut comprendre en outre des organes de fixation métalliques de ladite bride audit réservoir, tels que des vis et/ou des écrous, qui sont formés d'un seul tenant par surmoulage avec des oreilles de fixation de ladite bride.

On intègre de cette manière les organes de fixation et éventuellement de connexion diélectrique directement dans l'embout pour résoudre une quadruple problématique :
- la nécessité d'un bon contact électrique avec la structure,
- le fluage du thermoplastique sous fortes contraintes,
- la difficulté d'accès à certaines fixations, et
- la réduction du temps de montage.

En effet, la forme parfois complexe des divers embouts ou connecteurs existants (par exemple coudés à angle droit, ou d'autres géométries) peut empêcher l'accès frontal aux oreilles et donc aux vis ou écrous de fixation. Or, ce surmoulage selon l'invention des organes de fixation utilise avantageusement la technique d'injection pour intégrer directement des vis ou préférablement des écrous métalliques (pour la réparation par retaraudage), de préférence en titane ou en inox pour éviter les couples galvaniques avec les fibres de carbone contenues dans l'embout dans les oreilles de fixation en thermoplastique. Ce qui résout simultanément les problématiques de dissipation électrique, de risques de fluage et de temps de montage.

La technologie d'injection permet aussi d'y intégrer des inserts de fixation plus rapides que le vissage, par exemple des fixations « quart de tour », qui permettent encore de réduire le temps de montage des lignes carburant lors de l'assemblage final, tout en gardant les mêmes caractéristiques de maintien et de contact électrique.

Selon une autre caractéristique de l'invention, le dispositif de connexion peut comprendre en outre, à proximité de deux extrémités dudit embouts destinées à recevoir lesdits tubes, deux joints annulaires d'étanchéité électriquement conducteurs à base d'au moins un élastomère de préférence choisi dans le groupe constitué par les caoutchoucs silicones et fluorosilicones (caoutchoucs particulièrement avantageux pour leur tenue aux carburants, par exemple chargés de noir de carbone, de nanotubes de carbone et/ou d'autres particules conductrices) et qui sont montés au contact d'une face radialement interne dudit embout en étant rapportés dans deux gorges circonférentielles respectives desdits tubes ou bien surmoulés sur ces tubes, ces joints étant aptes à assurer la continuité électrique des tubes avec l'embout et avec ledit réservoir.

On notera que chaque joint élastomère électriquement conducteur ne pourrait pas être utilisé avec des embouts de connexion en aluminium car ceux-ci nécessitent un traitement antifriction ou anticorrosion qui rend leurs surfaces non électriquement conductrices. Dans la présente invention, ce type de joints peut être utilisé car chaque joint est en contact direct avec une matière électriquement conductrice qui ne présente pas de risque de corrosion, et permet alors d'assurer la continuité électrique des tubes avec l'embout et en outre, grâce aux oreilles de fixation de l'embout, avec la structure du réservoir de carburant adjacent.

Comme expliqué ci-dessous, on obtient ainsi la décharge électrostatique dans la structure de l'aéronef sans avoir besoin d'utiliser des tresses électriquement conductrices additionnelles comme par le passé.

Avantageusement, ledit embout peut être dépourvu de toute couche circonférentielle métallique.

Egalement avantageusement, ledit embout peut être pourvu de moyens de chauffage du fluide le parcourant qui sont surmoulés sur la paroi de l'embout. On utilise alors les propriétés de résistivité électrique de la pièce pour la rendre chauffante, ce qui est particulièrement intéressant lorsque les fluides transportés sont soumis au risque de gel (cas e.g. des avions longs courriers volant à une température extérieure proche de -55° C). Pour chauffer l'embout, on peut l'alimenter par une connexion électrique pouvant être ajoutée par surmoulage lors de l'injection de la pièce, étant précisé que le passage d'un faible courant élève la température de l'embout au-dessus du point de congélation du fluide véhiculé.

Une canalisation selon l'invention pour tuyauterie de transport de fluide dans un véhicule aérien ou spatial, la canalisation étant en particulier destinée à être montée dans chacune des ailes composites d'un avion pour transporter un carburant et comprenant deux tubes de préférence non métalliques reliés entre eux via une liaison coulissante et rotulante par un dispositif de connexion à embout composite à matrice thermoplastique, est caractérisée en ce que le dispositif de connexion est tel que défini précédemment et est dépourvu de tout connecteur métallique assemblé à l'embout pour la connexion de ce dernier aux tubes.

Avantageusement, la canalisation peut être dépourvue de toute tubulure électriquement isolante entre les tubes.

Egalement avantageusement, la canalisation peut être dépourvue de toute tresse électriquement conductrice solidaire de l'embout pour la connexion électrique de ce dernier auxdits tubes et audit réservoir, le dispositif de connexion comprenant, à proximité de deux extrémités de l'embout recevant les tubes, lesdits deux joints annulaires d'étanchéité électriquement conducteurs qui sont montés au contact d'une face radialement interne de l'embout en étant rapportés dans deux gorges circonférentielles respectives des tubes ou bien surmoulés sur ces tubes et qui assurent la continuité électrique des tubes avec l'embout et avec le réservoir.

On notera que le fait de se passer d'une telle tresse conductrice additionnelle permet de réduire de manière importante le poids, les temps de montage et les risques de mauvais montage des canalisations, et donc d'améliorer la sécurité du véhicule qui en est équipé, tel qu'un aéronef.

Un procédé de fabrication selon l'invention d'un dispositif de connexion tel que défini précédemment comprend :
a) un moulage de l'embout composite à matrice thermoplastique par injection séparée en des emplacements distincts d'un moule de ladite au moins une zone coudée ou incurvée et desdites deux portions droites qui s'étendent axialement de part et d'autre de cette zone, de préférence en utilisant un noyau rotatif à cinématique de sortie curviligne, et
b) un surmoulage ou un soudage de ces deux portions droites à cette zone coudée ou incurvée par l'intermédiaire de portées globalement cylindriques correspondant à ces portions droites.

On notera que ce procédé selon l'invention permet d'apporter une solution aux problèmes usuels des embouts thermoplastiques chargés de fibres, qui se posent au niveau des portées des joints et pour la réalisation des zones coudées (par exemple à 90°) sans aucun point anguleux dans l'intérieur du coude, pour éviter toute perte de charge ou cavitation dans l'écoulement du fluide transporté. En effet, pour être étanches, ces portées doivent être cylindriques et parfaitement lisses, donc sans ligne de soudure ou autre « couture » de moulage. Or, un usinage pour y remédier, vu la présence de fibres usinées en surface, provoquerait une usure prématurée des joints en endurance et augmenterait significativement le coût de fabrication des pièces.

Une solution pour avoir un conduit coudé à portées parfaitement lisses sans nécessité d'usinage pourrait consister à utiliser des noyaux complexes, combinant des tiroirs « à clefs » avec des noyaux à double mouvement (dégagement/rétraction) pour pouvoir les dépouiller. Mais cette solution est à la fois coûteuse en termes d'outillage et sa mécanisation peut être difficile du fait d'un encombrement des vérins ou autres mécanismes dans le moule.

On notera toutefois qu'il est possible en variante de ce procédé à deux étapes a) et b) selon l'invention de fabriquer cet embout directement, en une seule étape de moulage.

D'autres caractéristiques, avantages et détails de la présente invention ressortiront à la lecture de la description suivante de plusieurs exemples de réalisation de l'invention, donnés à titre illustratif et non limitatif, ladite description étant réalisée en référence avec les dessins joints, parmi lesquels :
la figure 1 est une vue schématique en section axiale d'une canalisation selon un exemple de l'invention à embout incurvé relié à deux tubes et en outre à une structure adjacente d'un aéronef,
la figure 2 est une vue schématique en section axiale d'une canalisation selon un autre exemple de l'invention à embout incurvé relié uniquement à deux tubes,
la figure 3 est une vue latérale en perspective d'un embout coudé selon un premier exemple de l'invention,
la figure 4 est une vue latérale en perspective d'un embout coudé selon un second exemple de l'invention,
la figure 5 est une vue latérale en perspective illustrant le démoulage d'un embout coudé selon un troisième exemple de l'invention,
la figure 6 est une vue de dessous en perspective de l'embout coudé obtenu par ce procédé de la figure 5 selon ce troisième exemple de l'invention, et
la figure 7 est une vue latérale en perspective d'un embout non coudé mais illustrant de manière schématique l'orientation des fibres de carbone dans un embout coudé ou incurvé selon l'invention.

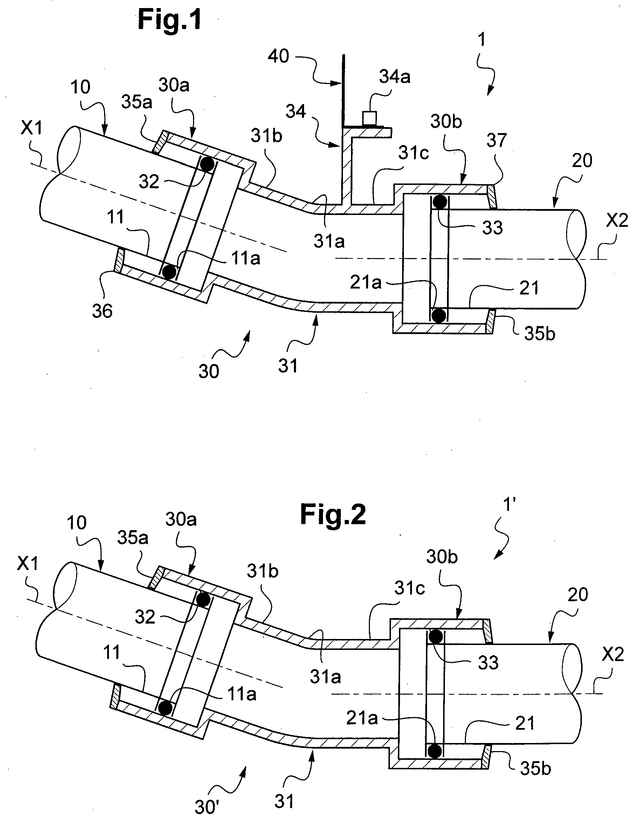

Une canalisation 1, 1' selon les exemples de l'invention visibles aux figures 1 et 2 est par exemple destinée à véhiculer un carburant d'aéronef, et elle est constituée de deux tubes 10 et 20 de préférence non métalliques et d'un dispositif de connexion 30, 30' qui les relie directement entre eux via des liaisons coulissantes et rotulantes en deux zones d'extrémités 30a et 30b respectives du dispositif 30, 30' et qui relie optionnellement en outre les tubes 10 et 20 à un réservoir de carburant 40 (voir figure 1). Ce dispositif 30, 30' est constitué dans ces deux cas d'un embout 31 moulé par injection de type composite à matrice thermoplastique présentant une zone centrale coudée ou incurvée 31a, et de deux joints annulaires d'étanchéité 32 et 33 électriquement conducteurs qui sont montés radialement entre et au contact de deux zones d'extrémités 11 et 30a, 21 et 30b respectives des tubes 10 et 20 et de l'embout 31.

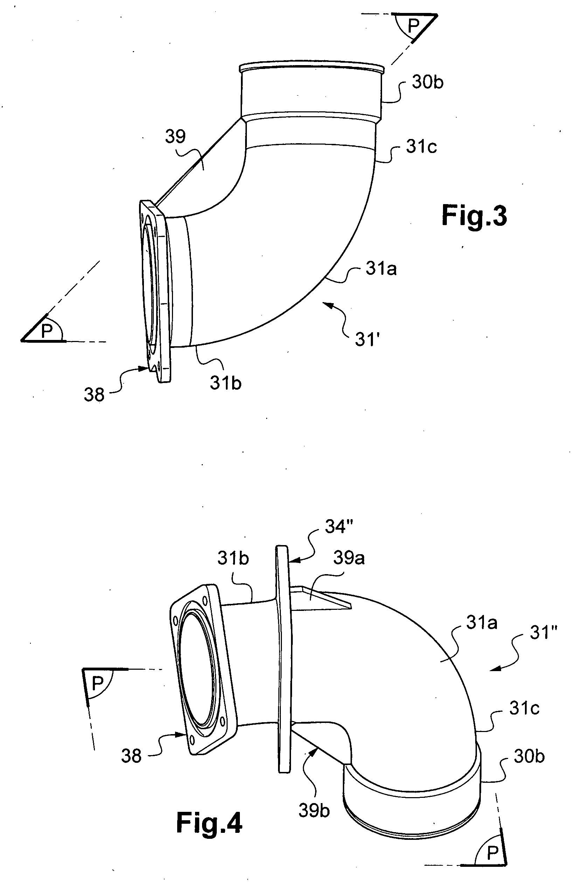

De manière générale, l'embout 31 selon l'invention présente un plan axial de joint de moulage P (visible aux figures 3, 4 et 6), qui correspond au plan longitudinal médian de l'embout 31 contenant les axes de symétries X1 et X2 respectifs de ses deux portions droites 31b et 31c situées axialement de part et d'autre de sa zone coudée ou incurvée 31a.

Ces joints conducteurs 32 et 33 assurent la continuité électrique des tubes 10 et 20 avec l'embout 31 et éventuellement avec le réservoir de carburant 40 dans l'exemple de la figure 1 par l'intermédiaire d'une bride 34 de fixation à ce réservoir 40 qui est prévue également conductrice, comme expliqué ci-après. Plus précisément, on voit que chaque tube 10, 20 est inséré axialement et radialement à l'intérieur d'une zone d'extrémité 30a, 30b de l'embout 31 correspondant à l'une de ses deux portions droites 31b et 31c. Dans ces exemples, chaque joint 32, 33 est rapporté ou surmoulé dans une gorge circonférentielle 11a, 21a d'une zone d'extrémité 11, 21 du tube 10, 20 correspondant. En d'autres termes, les tubes 10 et 20 selon les figures 1 et 2 sont emmanchés de manière étanche dans les deux portions droites 31b et 31c de l'embout 31 via ces joints 32 et 33, qui sont de préférence à base d'un caoutchouc silicone ou fluorosilicone chargé par exemple par du noir de carbone et/ou des nanotubes de carbone.

Il est également possible de surmouler un autre contact électrique 35a, 35b par exemple en chacune des deux extrémités 36 et 37 de l'embout 31. Chacun des deux contacts 35a, 35b visibles aux figures 1 et 2 est ainsi monté axialement en deçà du joint 32, 33 correspondant, et il relie radialement chaque tube 10, 20 à l'extrémité même 36, 37 de l'embout 31 en regard de ce tube 10, 20.

Dans l'exemple de la figure 1, l'embout 31 selon l'invention incorpore en outre à proximité de sa zone coudée 31a la bride radiale 34 pour fixer l'embout 31 à une structure du fuselage ou d'un réservoir de carburant 40 de l'aéronef. La bride 34 est formée d'un seul tenant avec l'embout 31, étant venue de moulage et se terminant par des moyens de mise à la masse électrique 34a à cette structure 40.

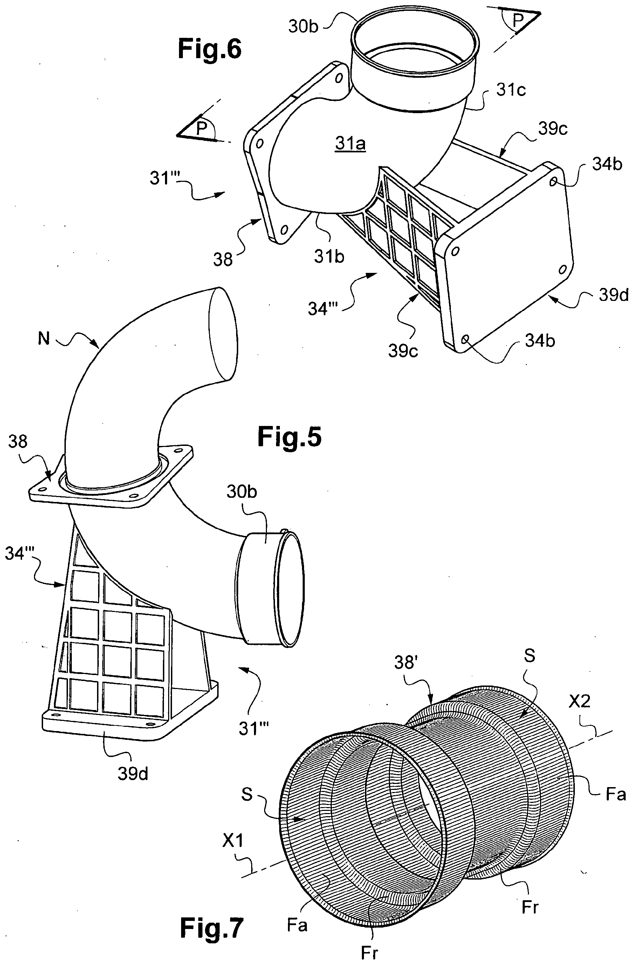

La figure 4 montre une variante de réalisation selon l'invention d'une bride radiale de fixation 34" qui s'étend ici sur toute la circonférence d'une portion droite 31a de l'embout coudé 31" et qui est par exemple destinée à être fixée à cette structure de réservoir 40. On réalise avantageusement cette bride 34" en lui adjoignant des organes de fixation métalliques pour la fixation de l'embout 31" au réservoir 40, tels que des vis et/ou des écrous formés d'un seul tenant par surmoulage avec des oreilles de fixation de la bride 34" (ces oreilles sont visibles à la variante de la figure 6). Comme expliqué plus haut, on notera que l'utilisation des joints conducteurs 32 et 33 combinée à ces organes de fixation de l'embout 31" au réservoir 40 permet de se passer de toute tresse électriquement conductrice reliant l'embout 31" aux tubes 10 et 20 et au réservoir 40.

Comme visible aux figures 3 à 6, l'une des extrémités de l'embout 31', 31", 31"' peut être munie à sa périphérie d'une bride auxiliaire 38 destinée à la connexion de l'embout 31', 31", 31'" au tube 10, 20 ou bien à un organe équipant la canalisation 1, 1', tel qu'une pompe, par exemple sur la même portion droite 31b que celle présentant éventuellement la bride 34" de fixation au réservoir 40 (voir figure 4). L'autre extrémité de l'embout 31', 31", 31'" axialement opposée à cette bride auxiliaire 38 peut présenter une zone élargie 30b formant un léger renflement d'extrémité.

Les tubes 10 et 20 sont par exemple réalisés en un matériau composite à matrice plastique et, selon l'invention, l'embout 31, 31', 31", 31'" est moulé par injection d'un matériau composite à matrice thermoplastique renforcée par des fibres de carbone discontinues « IM »ou « HM » orientées dans une direction globalement axiale et par des fibres et/ou particules électriquement isolantes (e.g. de verre ou d'aramide). La Demanderesse a notamment obtenu des résultats très avantageux en utilisant dans l'embout 31, 31', 31", 31'" selon l'invention des fibres de carbone de module intermédiaire (« IM ») en relation avec une matrice thermoplastique à base d'un polyamide, en particulier un PA12, et des fibres de carbone de haut module (« HM ») en relation avec une matrice thermoplastique à base d'un PEEK.

Selon l'invention, le dispositif de connexion 30, 30' et la canalisation 1,1' l'incorporant sont dépourvus de tout connecteur métallique assemblé à l'embout 31, 31', 31", 31"' pour la connexion de ce dernier aux tubes 10 et 20, la canalisation 1,1' étant en outre dépourvue de toute tubulure électriquement isolante entre tubes 10 et 20 consécutifs.

La figure 7 illustre schématiquement l'orientation des fibres de carbone selon l'invention, prévue majoritairement axiale le long de chaque surface cylindrique S (orientation Fa parallèle à l'axe longitudinal de symétrie X1, X2 de chaque surface S), et majoritairement radiale sur chaque collerette ou bride circonférentielle 38' (orientation Fr perpendiculaire à X1, X2).

Egalement selon l'invention et en référence aux figures 3 à 6, l'embout 31, 31', 31", 31"' incorpore des moyens 39, 39a et 39b, 39c adaptés pour le rigidifier d'un point de vue mécanique et vibratoire qui sont également venus de moulage avec l'embout 31, 31', 31", 31'" et sont donc constitués du même matériau que le reste de ce dernier (ces moyens de rigidification sont bien présents sur les embouts 31 des figures 1 et 2, bien que non visibles sur celles-ci car situés hors de la section axiale choisie pour ces figures).

Comme illustré dans le premier exemple de la figure 3, ces moyens de rigidification 39 peuvent comprendre une nervure 39 qui s'étend axialement et radialement en sa zone coudée 31a dans le plan axial de joint de moulage P de l'embout 31', sur la surface de courbure interne de cette zone coudée 31a. Dans cet exemple, cette nervure axiale 39 prend appui, d'une part, sur la bride auxiliaire d'extrémité 38 de l'embout 31' et, d'autre part, sur la zone d'extrémité élargie 30b axialement opposée de l'embout 31'.

Comme illustré dans le second exemple de la figure 4, les moyens de rigidification 39a et 39b peuvent comprendre, dans le plan de joint P de l'embout 31" :
- une première nervure axiale 39a sensiblement trapézoïdale sur la surface externe de la courbure qui part de cette surface avec une hauteur radiale croissante puis prend appui sur la bride de fixation 34" de l'embout 31" au réservoir 40, et
- une seconde nervure axiale 39b qui s'étend sur la surface interne de la courbure de manière analogue à la nervure 39 de la figure 3, en prenant appui, d'une part, sur la bride de fixation 34" et, d'autre part, juste en deçà de la zone d'extrémité élargie 30b axialement opposée de l'embout 31".

Comme illustré dans le troisième exemple de la figure 6, les moyens de rigidification 39c peuvent comprendre, de part et d'autre du plan de joint P de l'embout 31'", deux pattes 39c en forme de grilles qui s'étendent symétriquement l'une de l'autre par rapport à ce plan P en prenant appui sur la surface externe de la courbure et qui sont surmontées par une plaque de liaison 39d à la structure adjacente, telle que le réservoir 40 de carburant. La plaque 39d est munie d'organes de fixation métalliques pour la fixation de l'embout 31"' au réservoir 40, tels que des vis et/ou des écrous formés d'un seul tenant par surmoulage avec des oreilles de fixation 34b de la bride 34"'.

La figure 5 montre une phase d'un procédé de moulage utilisable à titre préférentiel pour fabriquer un embout 31'" non seulement selon la figure 6, mais de manière générale tout embout coudé ou incurvé 31, 31', 31" selon l'invention. Ce procédé consiste essentiellement à séparer la ou chaque zone coudée ou incurvée 31a des deux portions droites 31b et 31c adjacentes. On façonne cette zone coudée ou incurvée 31a par un moulage thermoplastique simple à mettre en oeuvre, avec un noyau rotatif N de type « sabre » qui est à cinématique de sortie curviligne, auquel on ajoute des portées cylindriques de chaque côté par surmoulage thermoplastique ou soudure thermoplastique. Ces portées sont alors aisées à réaliser sans ligne de soudure avec une très faible dépouille, qui est acceptable pour l'étanchéité en fonctionnement. On notera que les moules utilisés pour ce procédé sont relativement simples et donc peu onéreux.

## Revendications

1. Dispositif de connexion (30, 30') pour tuyauterie de transport de fluide d'un véhicule aérien ou spatial, le dispositif étant adapté pour relier deux tubes (10 et 20) entre eux et optionnellement en outre à un réservoir (40) dudit fluide, le dispositif comprenant un embout (31, 31', 31", 31"') moulé par injection qui présente au moins une zone coudée ou incurvée (31a) et qui est constitué d'un matériau composite à matrice thermoplastique renforcée par un système de renforcement comprenant des fibres de carbone, l'embout présentant un plan de joint de moulage (P) longitudinal médian, **caractérisé en ce que** lesdites fibres de carbone s'étendent de manière orientée le long de l'embout, lequel incorpore des moyens de rigidification mécanique et vibratoire (39, 39a et 39b, 39c) qui sont venus de moulage avec l'embout et qui s'étendent dans ledit plan de joint ou symétriquement par rapport à ce plan en ladite au moins une zone coudée ou incurvée et/ou à proximité immédiate de cette zone.

2. Dispositif de connexion (30, 30') selon la revendication 1, **caractérisé en ce que** ledit embout (31, 31', 31", 31'") comporte :
- deux portions droites (31b et 31c) qui s'étendent axialement de part et d'autre de ladite au moins une zone coudée ou incurvée (31a) et qui incorporent lesdites fibres de carbone majoritairement orientées dans la direction axiale (Fa) d'une surface globalement cylindrique (S) de chaque portion, et
- au moins une collerette (38') ou bride (38, 34, 34", 34"') par exemple pour fixer l'embout audit réservoir (40) qui est également venue de moulage avec l'embout et qui s'étend radialement de manière adjacente à ladite au moins une zone coudée ou incurvée, cette collerette ou bride incorporant lesdites fibres de carbone majoritairement orientées dans la direction radiale (Fr) autour de cette portion droite.

3. Dispositif de connexion (30, 30') selon la revendication 2, **caractérisé en ce que** lesdits moyens de rigidification mécanique et vibratoire (39, 39a et 39b, 39c) comprennent au moins une nervure (39, 39a et 39b) ou patte (39c) qui s'étend dans la direction axiale de l'embout (31', 31", 31"') et qui définit une surépaisseur radiale en ladite zone coudée ou incurvée (31a) de l'embout et/ou à proximité immédiate de cette zone.

4. Dispositif de connexion (30, 30') selon la revendication 3, **caractérisé en ce que** lesdits moyens de rigidification mécanique et vibratoire comprennent au moins une dite nervure (39, 39a et 39b) qui s'étend axialement dans ledit plan de joint (P) de l'embout (31', 31") et qui prend appui sur ladite bride (38, 34").

5. Dispositif de connexion (30, 30') selon la revendication 4, **caractérisé en ce que** ladite au moins une zone coudée ou incurvée (31a) présente une courbure par exemple à angle droit définissant une surface externe et une surface interne desdites portions droites (31b et 31c) adjacentes qui sont respectivement tournées vers l'extérieur et vers l'intérieur de la courbure, lesdits moyens de rigidification mécanique et vibratoire comprenant une première dite nervure axiale (39a) formant une surépaisseur radiale sur ladite surface externe et une seconde dite nervure axiale (39b) formant une surépaisseur radiale sur ladite surface interne.

6. Dispositif de connexion (30, 30') selon la revendication 3, **caractérisé en ce que** ladite au moins une zone coudée ou incurvée (31a) présente une courbure par exemple à angle droit définissant une surface externe et une surface interne desdites portions droites (31b et 31c) adjacentes qui sont respectivement tournées vers l'extérieur et vers l'intérieur de la courbure, lesdits moyens de rigidification mécanique et vibratoire comprenant deux dites pattes (39c) par exemple en forme de grilles qui s'étendent symétriquement l'une de l'autre par rapport audit plan de joint (P) de l'embout (31"') en prenant appui sur ladite surface externe et qui sont surmontées par une plaque (39d) de liaison audit réservoir (40).

7. Dispositif de connexion (30) selon une des revendications 2 à 6, **caractérisé en ce qu'**il comprend en outre des organes de fixation métalliques de ladite bride (34, 34", 34"') audit réservoir (40), tels que des vis et/ou des écrous, qui sont formés d'un seul tenant par surmoulage avec des oreilles de fixation (34b) de ladite bride.

8. Dispositif de connexion (30, 30') selon une des revendications précédentes, **caractérisé en ce que** ledit système de renforcement est présent dans ledit embout (31, 31', 31", 31'") selon une fraction massique comprise entre 10 % et 40 %, ce système comprenant :
- lesdites fibres de carbone, choisies dans le groupe constitué par les fibres de carbone de module intermédiaire (« IM ») ou de haut module (« HM »), et
- des fibres et/ou particules électriquement isolantes telles que des fibres de verre ou d'aramide, selon un ratio volumique fibres de carbone / fibres et/ou particules électriquement isolantes compris entre 30 % et 80 %.

9. Dispositif de connexion (30, 30') selon une des revendications précédentes, **caractérisé en ce que** ladite matrice thermoplastique est à base d'au moins un polymère choisi dans le groupe constitué par les polyamides tels que le PA 12, les polyaryléthercétones (PAEK), les polyétheréthercétones (PEEK), les polyéthercétonecétones (PEKK) et leurs alliages, cette matrice présentant une densité de préférence inférieure ou égale à 1,5.

10. Dispositif de connexion (30, 30') selon une des revendications précédentes, **caractérisé en ce qu'**il comprend en outre, à proximité de deux extrémités (36 et 37) dudit embout (31, 31', 31", 31"') destinées à recevoir lesdits tubes (10 et 20), deux joints annulaires d'étanchéité (32 et 33) électriquement conducteurs à base d'au moins un élastomère choisi dans le groupe constitué par les caoutchoucs silicones et fluorosilicones et qui sont montés au contact d'une face radialement interne dudit embout en étant rapportés dans deux gorges circonférentielles (11a et 21a) respectives desdits tubes ou bien surmoulés sur ces tubes, ces joints étant aptes à assurer la continuité électrique des tubes avec l'embout et avec ledit réservoir (40).

11. Dispositif de connexion (30, 30') selon la revendication 10, **caractérisé en ce que** ledit embout (31, 31', 31", 31"') est dépourvu de toute couche circonférentielle métallique.

12. Canalisation (1, 1') pour tuyauterie de transport de fluide dans un véhicule aérien ou spatial, la canalisation étant en particulier destinée à être montée dans chacune des ailes composites d'un avion pour transporter un carburant et comprenant deux tubes (10 et 20) de préférence non métalliques reliés entre eux via une liaison coulissante et rotulante par un dispositif de connexion (30, 30') à embout (31, 31', 31", 31"') composite à matrice thermoplastique, **caractérisée en ce que** le dispositif de connexion est tel que défini à l'une des revendications précédentes et est dépourvu de tout connecteur métallique assemblé à l'embout pour la connexion de ce dernier aux tubes.

13. Canalisation (1, 1') selon la revendication 12, **caractérisée en ce qu'**elle est dépourvue de toute tubulure électriquement isolante entre les tubes (10 et 20).

14. Canalisation (1, 1') selon la revendication 12 ou 13, **caractérisée en ce qu'**elle est dépourvue de toute tresse électriquement conductrice solidaire de l'embout (31, 31", 31'") pour la connexion électrique de ce dernier auxdits tubes (10 et 20) et audit réservoir (40), le dispositif de connexion (30) comprenant, à proximité de deux extrémités (36 et 37) de l'embout recevant les tubes, deux joints annulaires d'étanchéité (32 et 33) électriquement conducteurs à base d'au moins un élastomère choisi dans le groupe constitué par les caoutchoucs silicones et fluorosilicones et qui sont montés au contact d'une face radialement interne de l'embout en étant rapportés dans deux gorges circonférentielles (11a et 21a) respectives des tubes ou bien surmoulés sur ces tubes, ces joints assurant la continuité électrique des tubes avec l'embout et avec le réservoir.

15. Procédé de fabrication d'un dispositif de connexion (30, 30') selon une des revendications 1 à 11, **caractérisé en ce qu'**il comprend :
- un moulage dudit embout (31, 31', 31", 31"') composite à matrice thermoplastique par injection séparée, en des emplacements distincts d'un moule, de ladite au moins une zone coudée ou incurvée (31a) de l'embout et de deux portions droites (31b et 31c) de l'embout qui s'étendent axialement de part et d'autre de cette zone, de préférence en utilisant un noyau rotatif (N) à cinématique de sortie curviligne, et
- un surmoulage ou un soudage de ces deux portions à cette zone coudée ou incurvée par l'intermédiaire de portées globalement cylindriques correspondant à ces portions droites.

## Patentansprüche

1. Verbindungsvorrichtung (30, 30') für Flüssigkeitstransportrohrleitungen eines Luft- oder Raumfahrzeugs, wobei die Vorrichtung angepasst ist, um zwei Leitungsrohre (10 und 20) miteinander und wahlweise weiter mit einem Reservoir (40) der Flüssigkeit zu verbinden, wobei die Vorrichtung ein spritzgussgeformtes Ansatzstück (31, 31', 31", 31'") umfasst, das mindestens einen abgewinkelten oder gebogenen Bereich (31a) aufweist, und das aus einem durch ein Verstärkungssystem, umfassend Kohlenstofffasern, verstärkten thermoplastischen Matrix-Verbundmaterial besteht, wobei das Ansatzstück eine in Längsrichtung mittlere Formungsfügungsebene (P) aufweist, **dadurch gekennzeichnet, dass** sich die Kohlenstofffasern ausgerichtet entlang des Ansatzstücks erstrecken, welches mechanische und vibratorische Versteifungsmittel (39, 39a und 39b, 39c) einschließt, die aus der Formung mit dem Ansatzstück stammen und sich in der Fügungsebene oder bezogen auf diese Ebene, in dem mindestens einen abgewinkelten oder gebogenen Bereich und/oder in der unmittelbaren Nähe dieses Bereichs, symmetrisch erstrecken.

2. Verbindungsvorrichtung (30, 30') nach Anspruch 1, **dadurch gekennzeichnet, dass** das Ansatzstück (31, 31', 31", 31'") Folgendes beinhaltet:
- zwei gerade Abschnitte (31b und 31c), die sich axial auf beiden Seiten des mindestens einen abgewinkelten oder gebogenen Bereichs (31a) erstrecken und die die hauptsächlich in der axialen Richtung (Fa) einer global zylindrischen Oberfläche (S) von jedem Abschnitt ausgerichteten Kohlenstofffasern einschließen, und
- mindestens einen Kragen (38') oder Flansch (38, 34, 34", 34"'), zum Beispiel zum Fixieren des Ansatzstücks am Reservoir (40), das ebenfalls aus der Formung mit dem Ansatzstück, das sich benachbart radial zu dem mindestens einen abgewinkelten oder gebogenen Bereich erstreckt, stammt, wobei dieser Kragen oder Flansch die hauptsächlich in der radialen Richtung (Fr) um diesen geraden Abschnitt ausgerichteten Kohlenstofffasern einschließt.

3. Verbindungsvorrichtung (30, 30') nach Anspruch 2, **dadurch gekennzeichnet, dass** die mechanischen und vibratorischen Versteifungsmittel (39, 39a und 39b, 39c) mindestens eine Rippe (39, 39a und 39b) oder Lasche (39c) umfassen, die sich in der axialen Richtung des Ansatzstücks (31', 31", 31'") erstreckt und die eine radiale Verdickung in dem abgewinkelten oder gebogenen Bereich (31a) des Ansatzstücks und/oder in der unmittelbaren Nähe dieses Bereichs definiert.

4. Verbindungsvorrichtung (30, 30') nach Anspruch 3, **dadurch gekennzeichnet, dass** die mechanischen und vibratorischen Versteifungsmittel mindestens eine Rippe (39, 39a und 39b) umfassen, die sich axial in der Fügungsebene (P) des Ansatzstücks (31', 31") erstreckt und die an dem Flansch (38, 34") anliegt.

5. Verbindungsvorrichtung (30, 30') nach Anspruch 4, **dadurch gekennzeichnet, dass** der mindestens eine abgewinkelte oder gebogene Bereich (31a) eine Krümmung, zum Beispiel im rechten Winkel, aufweist, die eine externe Oberfläche und eine interne Oberfläche der benachbarten geraden Abschnitte (31b und 31c) definiert, die jeweils zum Äußeren und zum Inneren der Krümmung gedreht sind, wobei die mechanischen und vibratorischen Versteifungsmittel eine erste axiale Rippe (39a), die eine radiale Verdickung auf der externen Oberfläche bildet, und eine zweite axiale Rippe (39b), die eine radiale Verdickung auf der internen Oberfläche bildet, umfassen.

6. Verbindungsvorrichtung (30, 30') nach Anspruch 3, **dadurch gekennzeichnet, dass** der mindestens eine abgewinkelte oder gebogene Bereich (31a) eine Krümmung, zum Beispiel im rechten Winkel, aufweist, die eine externe Oberfläche und eine interne Oberfläche der benachbarten geraden Abschnitte (31b und 31c) definiert, die jeweils zum Äußeren und zum Inneren der Krümmung gedreht sind, wobei die mechanischen und vibratorischen Versteifungsmittel zwei Laschen (39c), zum Beispiel in Form von Gittern, aufweisen, die sich bezogen auf die Fügungsebene (P) des Ansatzstücks (31''') zueinander symmetrisch erstrecken und sich an der externen Oberfläche anlegen und die durch eine Verbindungsplatte (39d) auf dem Reservoir (40) überdeckt sind.

7. Verbindungsvorrichtung (30) nach einem der Ansprüche 2 bis 6, **dadurch gekennzeichnet, dass** sie weiter metallische Fixierungsorgane des Flanschs (34, 34", 34"') an dem Reservoir (40), wie Schrauben und/oder Muttern, umfasst, die in integrierter Weise durch Umspritzung mit Fixierlaschen (34b) des Flansches gebildet werden.

8. Verbindungsvorrichtung (30, 30') nach einem der vorstehenden Ansprüche, **dadurch gekennzeichnet, dass** das Verstärkungssystem in dem Ansatzstück (31, 31', 31", 31"') in einem Masseanteil im Bereich von 10 % und 40 % vorliegt, wobei dieses System Folgendes umfasst:
- die Kohlenstofffasern, ausgewählt aus der Gruppe, bestehend aus Kohlenstofffasern mit Zwischenmodul ("IM") oder Hochmodul ("HM"), und
- Fasern und/oder elektrisch isolierende Partikel, wie Glas- oder Aramidfasern, nach einem Volumenverhältnis zwischen Kohlenstofffasern/elektrisch isolierenden Fasern und/oder Partikeln im Bereich zwischen 30 % und 80 %.

9. Verbindungsvorrichtung (30, 30') nach einem der vorstehenden Ansprüche, **dadurch gekennzeichnet, dass** die thermoplastische Matrix auf mindestens einem Polymer basiert, ausgewählt aus der Gruppe, bestehend aus Polyamiden wie PA 12, Polyaryletherketonen (PAEK), Polyetheretherketonen (PEEK), Poletherketonketonen (PEKK) und deren Legierungen, wobei diese Matrix eine Dichte von vorzugsweise kleiner als oder gleich 1,5 aufweist.

10. Verbindungsvorrichtung (30, 30') nach einem der vorstehenden Ansprüche, **dadurch gekennzeichnet, dass** sie weiter in der Nähe von zwei Enden (36 und 37) des Ansatzstücks (31, 31', 31", 31"'), die dazu bestimmt sind, die Leitungsrohre (10 und 20) aufzunehmen, zwei ringförmige, elektrisch leitende Dichtungen (32 und 33) auf Basis von mindestens einem Elastomer umfasst, ausgewählt aus der Gruppe, bestehend aus Silikon- und Fluorsilikonkautschuken, und die in Kontakt mit einer radial internen Fläche des Ansatzstücks montiert sind, indem sie in zwei jeweilige umlaufende Aussparungen (11a und 21a) der Leitungsrohre beigebracht werden oder auf diesen Leitungsrohren umspritzt sind, wobei diese Dichtungen imstande sind, die elektrische Kontinuität der Leitungsrohre mit dem Ansatzstück und mit dem Reservoir (40) zu gewährleisten.

11. Verbindungsvorrichtung (30, 30') nach Anspruch 10, **dadurch gekennzeichnet, dass** das Ansatzstück (31, 31', 31", 31'") frei von jeder umlaufenden metallischen Schicht ist.

12. Rohr (1, 1') für Flüssigkeitstransportrohrleitungen in einem Luft- oder Raumfahrzeug, wobei das Rohr insbesondere dazu bestimmt ist, in jedem der Verbundmaterialflügel eines Flugzeugs zum Transportieren eines Treibstoffs montiert zu werden, und zwei miteinander durch eine Gleitverbindung und durch eine Verbindungsvorrichtung (30, 30') am Verbundmaterial-Ansatzstück (31, 31', 31", 31"') mit thermoplastischer Matrix verknüpfte, vorzugsweise nicht metallische Leitungsrohre (10 und 20) umfassend, **dadurch gekennzeichnet, dass** die Verbindungsvorrichtung nach einem der vorstehenden Ansprüche definiert ist und frei ist von jedem am Ansatzstück angebauten metallischen Verbindungsstück zur Verbindung von Letzterem mit den Rohren.

13. Rohr (1, 1') nach Anspruch 12, **dadurch gekennzeichnet, dass** es frei ist von jedem elektrisch isolierenden Krümmer zwischen den Rohren (10 und 20).

14. Rohr (1, 1') nach Anspruch 12 oder 13, **dadurch gekennzeichnet, dass** es frei von jedem fest mit dem Ansatzstück (31, 31", 31"') verbundenen elektrisch leitenden Geflecht, zur elektrischen Verbindung von Letzterem mit den Leitungsrohren (10 und 20) und dem Reservoir (40) ist, wobei die Verbindungsvorrichtung (30) in der Nähe von zwei Enden (36 und 37) des die Leitungsrohre aufnehmenden Ansatzstücks zwei ringförmige elektrisch leitende Dichtungungen (32 und 33) auf Basis von mindestens einem Elastomer umfasst, ausgewählt aus der Gruppe, bestehend aus Silikon- und Flluorsilikonkautschuken, und die in Kontakt mit einer radial internen Fläche des Ansatzstücks montiert sind und in zwei umlaufenden Aussparungen (11a und 21a) der jeweiligen Leitungsrohre beigebracht sind oder auf diesen Leitungsrohren umspritzt sind, wobei diese Dichtungen die elektrische Kontinuität der Leitungsrohre mit dem Ansatzstück und mit dem Reservoir gewährleisten.

15. Verfahren zur Herstellungsverfahren einer Verbindungsvorrichtung (30, 30') nach einem der Ansprüche 1 bis 11, **dadurch gekennzeichnet, dass** es Folgendes umfasst:
- einen Abguss des Verbundmaterialansatzstücks (31, 31', 31", 31'") mit thermoplastischer Matrix durch separate Einspritzung in unterschiedliche Anordnungen einer Form, wobei der mindestens eine abgewinkelte oder gebogene Bereich (31a) des Ansatzstücks und der zwei geraden Abschnitte (31b und 31c) des Ansatzstücks, die sich auf beiden Seiten axial von diesem Bereich erstrecken, vorzugsweise unter Verwendung eines drehbaren Knotens (N) mit krummliniger Ausgangskinematik, und
- eine Umspritzung oder eine Verschweißung dieser beiden Abschnitte an diesen abgewinkelten oder gebogenen Bereich mittels global zylindrischer Tragweiten, die diesen geraden Abschnitten entsprechen.

## Claims

1. A connection device (30, 30') for fluid transmission piping of an aircraft or spacecraft, the device being suitable for connecting two tubes (10 and 20) to one another and optionally furthermore to a fluid tank (40), the device comprising an injection-molded tip (31, 31', 31", 31'") that has at least one bent or curved zone (31a) and that is made from a composite material with a thermoplastic matrix reinforced by a reinforcing system comprising carbon fibers, the tip having a median longitudinal mold parting plane (P), **characterized in that** said carbon fibers extend in an oriented manner along the tip, which incorporates mechanical and vibrational stiffening means (39, 39a and 39b, 39c) that are molded to be integral with the tip and extend in said parting plane or symmetrically relative to that plane in said at least one bent or curved zone and/or in the immediate vicinity of that zone.

2. The connection device (30, 30') according to claim 1, **characterized in that** said tip (31, 31', 31", 31'") comprises:
- two straight portions (31b and 31c) that extend axially on either side of said at least one bent or curved zone (31a) and which incorporate said carbon fibers, the majority of which are oriented in the axial direction (Fa) of a globally cylindrical surface (S) of each portion, and
- at least one collar (38') or flange (38, 34, 34", 34"') for example fastening the tip to said tank (40) that is also molded to be integral with the tip and that extends radially so as to be adjacent to said at least one bent or curved zone, this collar or flange incorporating said carbon fibers, the majority of which are oriented in the radial direction (Fr) around the straight portion.

3. The connection device (30, 30') according to claim 2, **characterized in that** said mechanical and vibrational stiffening means (39, 39a and 39b, 39c) comprise at least one rib (39, 39a and 39b) or tab (39c) that extends in the axial direction of the tip (31', 31", 31'") and defines a radial overthickness in said at least one bent or curved zone (31a) and/or in the immediate vicinity of the zone.

4. The connection device (30, 30') according to claim 3, **characterized in that** said mechanical and vibrational stiffening means comprise at least one said rib (39, 39a and 39b) that extends axially in said parting plane (P) of the tip (31', 31") and that bears on said flange (38, 34").

5. The connection device (30, 30') according to claim 4, **characterized in that** said at least one bent or curved zone (31a) has a curvature for example with a right angle defining an outer surface and an inner surface of the adjacent straight portions (31b and 31c) that are respectively turned toward the outside and the inside of the curve, said mechanical and vibrational stiffening means comprising a first so-called axial rib (39a) forming a radial overthickness on said outer surface and/or a second so-called axial rib (39b) forming a radial overthickness on said inner surface.

6. The connection device (30, 30') according to claim 3, **characterized in that** said at least one bent or curved zone (31a) has a curvature for example with a right angle defining an outer surface and an inner surface of the adjacent straight portions (31b and 31c) that are respectively turned toward the outside and the inside of the curve, said mechanical and vibrational stiffening means comprising two said tabs (39c) for example in the form of grates that extend symmetrically to one another relative to said parting plane (P) of the tip (31") while bearing on said outer surface of the curve and that are topped by a connecting plate (39d) to said tank (40).

7. The connection device (30) according to one of claims 2 to 6, **characterized in that** it further comprises metal fastening members for fastening said flange (34, 34", 34'") to said tank (40), such as screws and/or nuts, which are formed in a single piece by overmolding with fastening lugs (34b) of said flange.

8. The connection device (30, 30') according to one of the preceding claims, **characterized in that** said reinforcing system is present in said tip (31, 31', 31", 31"') according to a mass fraction comprised between 10% and 40%, this system comprising:
- said carbon fibers, chosen in the group consisting of those with an intermediate modulus ("IM") or high modulus ("HM"), and
- electrically insulating fibers and/or particles such as glass or aramid fibers, according to a carbon fiber / electrically insulating fibers and/or particles volume ratio comprised between 30% and 80%.

9. The connection device (30, 30') according to one of the preceding claims, **characterized in that** said thermoplastic matrix has a base of at least one polymer chosen from the group made up of polyamides such as PA 12, polyaryletherketones (PAEK), polyether ether ketones (PEEK), polyetherketoneketones (PEKK) and their alloys, this matrix having a density preferably lower than or equal to 1.5.

10. The connection device (30, 30') according to one of the preceding claims, **characterized in that** it further comprises, near two ends (36 and 37) of said tip (31, 31', 31", 31'") intended to receive said tubes (10 and 20), two electrically conductive annular sealing gaskets (32 and 33) with a base of at least one elastomer chosen from the group made up of silicone and fluorosilicone rubbers and that are mounted in contact with a radially inner face of said tip while being attached in two respective circumferential grooves (11a and 21a) of said tubes or overmolded on those tubes, these joints being able to ensure the electrical continuity of the tubes with the tip and the tank (40).

11. The connection device (30, 30') according to claim 10, **characterized in that** said tip (31, 31', 31", 31"') has no metal circumferential layer.

12. A pipe (1, 1') for fluid transmission piping in an aircraft or spacecraft, the pipe in particular being intended to be mounted in each of the composite wings of an airplane to transport a fuel and comprising two tubes (10 and 20) that are preferably nonmetallic and are connected to one another via a sliding and rolling link by a connecting device (30, 30') with a thermoplastic matrix composite tip(31, 31', 31", 31'"), **characterized in that** the connecting device is as defined in one of the preceding claims and has no metal connector assembled to the tip for the connection of the latter to the tubes.

13. The pipe (1, 1') according to claim 12, **characterized in that** it has no electrically insulating tubing between the tubes (10 and 20).

14. The pipe (1, 1') according to claim 12 or 13, **characterized in that** it has no electrically conductive braid secured to the tip (31, 31", 31'") for the electrical connection of the latter to said tubes (10 and 20) and said tank (40), the connection device (30) comprising, near two ends (36 and 37) of the tip receiving the tubes, said two electrically conductive annular sealing gaskets (32 and 33) with a base of at least one elastomer chosen from the group made up of silicone and fluorosilicone rubbers and that are mounted in contact with a radially inner face of the tip while being attached in two respective circumferential grooves (11a and 21a) of the tubes or overmolded on these tubes and that ensure the electrical continuity of the tubes with the tip and the tank.

15. A method for manufacturing a connection device (30, 30') according to one of claims 1 to 11, **characterized in that** it comprises:
- injection molding the thermoplastic matrix composite tip (31, 31', 31", 31"') in separate locations of a mold of said at least one bent or curved zone (31a) and said two straight portions (31b and 31c) of the tip that extend axially on either side of that zone, preferably using a rotating core (N) with curved output kinematics, and
- overmolding or welding these two straight portions to this bent or curved zone by means of globally cylindrical steps corresponding to these straight portions.
